# EUROPÄISCHE PATENTANMELDUNG

(11) **EP 1 166 750 A2**
(43) Veröffentlichungstag der Anmeldung: **02.01.2002**
(21) Anmeldenummer: 01113595.1
(22) Anmeldetag: 15.06.2001
(51) Int. Cl.: A61K 7/13

(54) **Haarfärbemittel**

(30) Priorität: 19.06.2000 DE 10030096
(71) Anmelder: GOLDWELL GmbH, 64280 Darmstadt (DE)
(72) Erfinder: Nöcker, Bernd, Dr., 64372 Ober-Ramstadt (DE); Theis, Heinz, Dr., 64354 Reinheim (DE); Kure, Naohisa, Dr., Sumida-ku, Tokyo 131 (JP)

(57) **Zusammenfassung**

Ein haarschonenden Haarfärbemittel mit guten Färbeeigenschaften und gleichzeitiger haarkonditionierender Wirkung enthält
a) mindestens einen direktziehenden Haarfarbstoff, und
b) mindestens ein Organopolysiloxan, bei dem mindestens ein Siliciumatom über eine Alkylengruppe, die ein Heteroatom, insbesondere ein Stickstoffatom, aufweist, mit einem Poly-(N-acylalkylenimin) mit Einheiten der Formel I 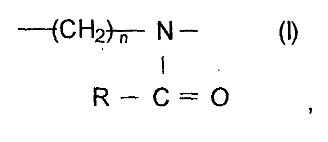
wobei n eine Zahl von 1 bis 5 und R Wasserstoff, eine C₁-C₁₂-Alkyl-, Cyclo-alkyl-, Aralkyl- oder Arylgruppe bedeuten, verbunden ist.

## Beschreibung

Die Erfindung betrifft ein Haarfärbemittel auf wäßriger Basis, das mindestens einen direktziehenden Haarfarbstoff enthält.
Derartige Haarfärbemittel sind seit langem bekannt.
Sie enthalten meistens mehrere anionische oder kationische direktziehende Farbstoffe und bedürfen, im Gegensatz zu den permanenten Haarfärbemitteln auf Basis von Oxidationsfarbstoffvorprodukten, keiner vorherigen Entwicklung mit Oxidationsmitteln.

Diese direktfärbenden Zusammensetzungen werden entweder zusammen mit oberflächenaktiven Mitteln als sogenannte Tönungsshampoos appliziert, oder als Lotionen, Emulsionen oder verdickte Lösungen, d.h., Gele, auf das Haar aufgebracht.

Diese direktziehenden Haarfärbemittel sind hinsichtlich ihrer Farbstabilität und insbesondere -intensität sowie der Gleichmäßigkeit der Färbung und des Farbglanzes noch verbesserungsfähig.

Die Erfindung geht daher von der Aufgabenstellung aus, ein Haarfärbemittel auf wäßriger Basis zu schaffen, das als Lotion, Emulsion, Lösung, Gel, Suspension bzw. auch, unter Zusatz von Treibmitteln, als Aerosolzusammensetzung auf das Haar aufgebracht werden kann, das optimale haarkonditionierende Eigenschaften aufweist, und sowohl mit anionischen als auch kationischen direktziehenden Haarfarbstoffen ausgezeichnete färberische Eigenschaften zeigt.

Die Lösung dieser Aufgabe besteht darin, einem solchen Mittel ein Organopolysiloxan, bei dem mindestens ein Siliciumatom über eine Alkylengruppe, die ein Heteroatom, insbesondere ein Stickstoffatom, aufweist, mit einem Poly-(N-acylalkylenimin) mit Einheiten der Formel I 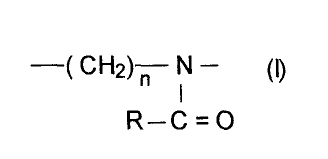 wobei n eine Zahl von 1 bis 5 und R Wasserstoff, eine C₁-C₁₂-Alkyl- oder-Cycloalkyl-, -Aralkyl- oder -Arylgruppe bedeuten, verbunden ist, vorzugsweise in einer Menge von 0,01 bis 5 Gew.-%, insbesondere 0,05 bis 2,5 Gew.-%, bezogen auf die Gesamtmenge des Mittels, Bevorzugte Organopolysiloxan-Polymere sind solche, wie sie in der EP-A 640 643 beschrieben sind, insbesondere gegebenenfalls quaternierte Aminoalkyl-, insbesondere Aminopropyl-dimethylpolysiloxan/Polyethyloxazolin-Copolymerisate der Formel 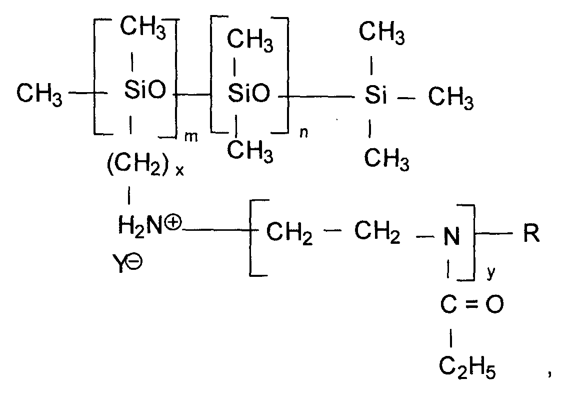 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R eine C₁-C₁₂-Alkyl- oder Arylgruppe, insbesondere eine Methyl-, Ethyl- oder Benzylgruppe, und Y⁻ ein Anion darstellen.

Die Herstellung der erfindungsgemäß bevorzugt zum Einsatz gelangenden Aminoalkyldimethylpolysiloxan/Polyethyloxazolin-Pfropfcopolymerisate ist in der bereits erwähnten EP-A 640 643 beschrieben und erfolgt entsprechend dem folgenden Schema: 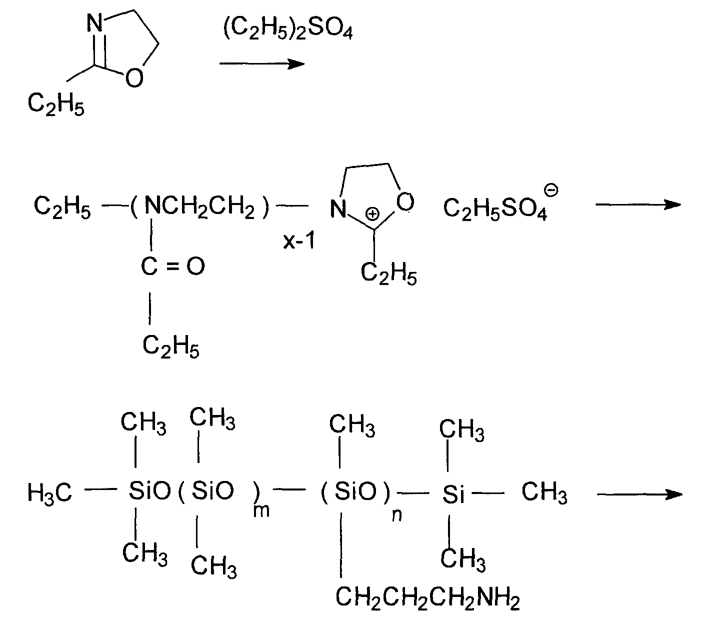 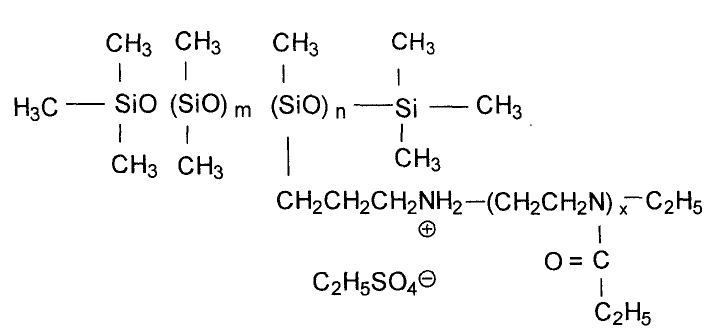 Das Anion Y⁻ der allgemeinen Formel kann selbstverständlich auch ein anderes als das Ethylsulfat-Anion des obengenannten Beispiels sein, d.h. die Quaternierung kann auch mit Methylchlorid, Dimethylsulfat, Benzylchlorid, Dodecylbromid etc. erfolgen.

Ein besonders bevorzugtes Pfropfcopolymerisat der dargestellten Art weist ein Gesamtmolekulargewicht von etwa 50 000 bis etwa 500 000, vorzugsweise etwa 80 000 bis etwa 300 000, insbesondere etwa 100 000 Dalton auf, wobei das Molgewicht des Oxazolin-Segments etwa 2 500 bis etwa 7 500, vorzugsweise etwa 4 000 bis etwa 6 000, insbesondere etwa 5 000 Dalton/Segment beträgt, d.h. der Molanteil bei 20 Einheiten/Molekül liegt. Der bevorzugte Si-Gehalt beträgt, entsprechend der Elementaranalyse, etwa 50 %.

Besonders geeignet sind die unter den Bezeichnungen A-1, A-2 und A-3 auf den Seiten 12 bis 13 der EP-A 640 643 beschriebenen Organopolysiloxane.
Der Anteil der Pfropfcopolymerisate in den erfindungsgemäßen Haarbehandlungsmitteln liegt bei etwa 0,05 bis 5, vorzugsweise 0,1 bis 2,5, insbesondere 0,5 bis 1,5 Gew.-% der Zusammensetzung.

Dieses Polymer ist sowohl mit kationischen als auch mit anionischen Farbstoffen ausgezeichnet kompatibel; die Zusammensetzungen sind über längere Zeit selbst bei sauren pH-Werten lagerstabil und ergeben intensive, glänzende, gleichmäßige Haarfärbungen.
Zusätzlich wird auch noch eine haarkonditionierende Wirkung, insbesondere eine verbesserte Naß- und Trockenkämmbarkeit, lockerer Griff, Volumen und Spannkraft erzielt; jedwede beim wiederholten Färben des Haares zuweilen auftretende Rauhigkeit des Haares wird vermieden.

Der Anteil der direktziehenden Farbstoffe in den erfindungsgemäßen Zusammensetzungen ist variabel und liegt zwischen etwa 0,005 bis etwa 5, vorzugsweise 0,01 bis 2,5, insbesondere 0,1 bis 1 Gew.-% des Mittels.

Als direktziehende Haarfarbstoffe können im Prinzip alle für diesen Zweck vorgeschlagenen kationischen Farbstoffe verwendet werden.

Bevorzugt sind die sogenannten "Arianor"-Farbstoffe; vgl. K. Schrader, "Grundlagen und Rezepturen der Kosmetika", 2. Aufl. (1989), S. 811.

### Besonders geeignete basische (kationische) Farbstoffe sind:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 12 | C.I.-No. 48,070; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719. |

Selbstverständlich ist auch die Verwendung entsprechender direktziehender Pflanzenfarbstoffe oder auch anionischer (saurer) direktziehender Haarfarbstoffe möglich.

Diese werden üblicherweise ebenfalls in einer Menge von etwa 0,005 bis etwa 5, vorzugsweise etwa 0,05 bis etwa 2,5, insbesondere etwa 0,1 bis etwa 1 Gew.-%, berechnet auf die Gesamtzusammensetzung des Mittels, das als Lösung, Dispersion, Emulsion, Gel oder Aerosolpräparat zur direkten Anwendung vorliegt, eingesetzt.

### Als geeignete anionische Farbstoffe können beispielsweise Verwendung finden:

| | |
|---|---|
| Acid Black 1, | C.I.-No. 20,470; |
| Acid Blue 1, | C.I.-No. 42,045; |
| Food Blue 5, | C.I.-No. 42,051; |
| Acid Blue 9, | C.I.-No. 42,090; |
| Acid Blue 74, | C.I.-No. 73,015; |
| Acid Red 18, | C.I.-No. 16,255; |
| Acid Red 27, | C.I.-No. 16,185; |
| Acid Red 87, | C.I.-No. 45,380; |
| Acid Red 92, | C.I.-No. 45,410; |
| Acid Orange 7, | C.I.-No. 15,510; |
| Acid Violet 43, | C.I.-No. 60,730; |
| Acid Yellow 1, | C.I.-No. 10,316; |
| Acid Yellow 23, | C.I.-No. 19,140; |
| Acid Yellow 3, | C.I.-No. 47,005; |
| Food Yellow No. 8, | C.I.-No. 14,270; |
| D&C Brown No. 1, | C.I.-No. 20,170 |
| D&C Green No. 5, | C.I.-No. 61,570; |
| D&C Orange No. 4, | C.I.-No. 15,510; |
| D&C Orange No. 10, | C.i.-No 45,425:1; |
| D&C Orange No. 11, | C.I.-No. 45,425; |
| D&C Red No. 21, | C.I.-No. 45,380:2; |
| D&C Red No. 27, | C.I.-No. 45,410:1; |
| D&C Red No. 33, | C.I.-No. 17,200; |
| D&C Yellow No. 7, | C.I.-No. 45,350:1; |
| D&C Yellow No. 8, | C.I.-No. 45,350; |
| FD&C Red No. 4, | C.I.-No. 14,700; |
| FD&C Yellow No. 6, | C.I.-No. 15,985. |

Auch pflanzliche Farbstoffe können allein oder in Kombination mit synthetischen Direktziehern Verwendung finden, beispielsweise Henna (rot oder schwarz), Alkannawurzel, Laccainsäure (Stocklack), Indigo, Blauholzpulver, Krappwurzel- und Rhabarberwurzelpulver, etc.

Das erfindungsgemäße Haarfärbemittel kann auch oberflächenaktive Substanzen enthalten, falls es sich um ein Tönungsshampoo handelt, sind diese ohnehin obligatorisch.

Solche können anionisch, nichtionisch, kationisch oder amphoter bzw. zwitterionisch sein.

Bevorzugt sind für Nichttönungsshampoos nichtionische und kationische Tenside in einer Menge zwischen etwa 0,5 und etwa 5 Gew.-%, berechnet auf die Gesamtzusammensetzung.

Geeignete nichtionische Tenside sind Verbindungen aus der Klasse der Alkylpolyglucoside mit der allgemeinen Formel

R-0-(CH₂CH₂0)ₙ-Zₓ,

worin R eine Alkylgruppe mit 8 bis 20, vorzugsweise 10 bis 14 Kohlenstoffatomen, Zₓ einen Saccharidrest mit 5 bis 6 Kohlenstoffatomen, n, eine Zahl von 0 bis 10, und x eine Zahl zwischen 1 und 5, vorzugsweise 1,1 bis 2,5 bedeuten.

Weitere geeignete nichtionische Tenside in den erfindungsgemäßen Mitteln sind C₁₀-C₂₂-Fettalkoholethoxylate.

Besonders geeignete C₁₀-C₂₂-Fettalkoholether sind die unter den Trivialnamen "Laureth", "Myristeth", "Oleth", Ceteth", "Deceth", "Steareth" und "Ceteareth" nach der CTFA-Nomenklatur mit Anfügung der Zahl der Ethylenoxid-Moleküle bezeichneten Alkylpolyglykolether, z.B. "Laureth-16":

Der durchschnittliche Ethoxylierungsgrad liegt dabei zwischen etwa 2,5 und etwa 25, vorzugsweise etwa 10 und etwa 20.

Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics® " im Verkehr sind.

Weitere zusätzlich einsetzbare Tenside sind Aminoxide.
Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl-oder ethylaminoxide, C₁₂-C₁₈-Alkyldi(hydroxyethyl)- oder -(hydroxypropyl)aminoxide, oder auch Aminoxide mit Ethylenoxid- und/oder Propylenoxidgruppen in der Alkylkette.

Geeignete Aminoxide sind beispielsweise unter den Bezeichnungen "Ammonyx® ", "Aromox® ", oder "Genaminox® " im Handel.

Weitere fakultative Tensidbestandteile sind Fettsäuremono- und dialkanolamide, wie Cocosfettsäuremonoethanolamid und Myristinfettsäuremonoisopropanolamid.

Geeignete amphotere bzw. zwitterionische Tenside sind insbesondere die verschiedenen bekannten Betaine wie Fettsäureamidoalkylbetaine und Sulfobetaine, beispielsweise Laurylhydroxysulfobetain; auch langkettige Alkylaminosäuren wie Cocoaminoacetat, Cocoaminopropionat und Natrium-cocoamphopropionat und -acetat haben sich als geeignet erwiesen.

In einzelnen können Betaine der Struktur 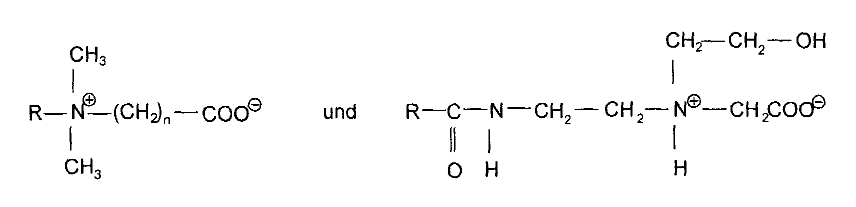 wobei R eine C₈-C₁₈-Alkylgruppe und n 1 bis 3 bedeuten,

Sulfobetaine der Struktur wobei R eine C₈-C₁₈-bedeuten, 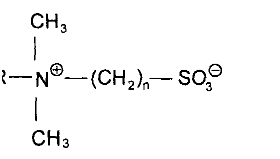 Alkylgruppe und n 1 bis 3 . 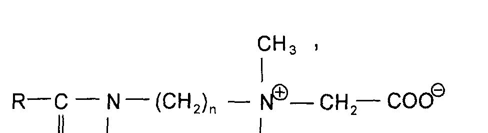 wobei R eine C₈-C₁₈-Alkylgruppe und n 1 bis 3 bedeuten, verwendet werden.

Bevorzugt sind Fettsäureamidoalkylbetaine, insbesondere Cocoamidopropylbetain, und Cocoamphoacetat und-propionat, insbesondere deren Natriumsalze. Besonders bevorzugt sind Gemische aus Cocoamidopropylbetain und Cocoamphoacetat, insbesondere im Gewichtsverhältnis 3:1 bis 1:3, vor allem 2:1 bis 1:1.

Geeignete kationische Tenside sind beispielsweise langkettige quaternäre Ammoniumverbindungen, die allein oder im Gemisch miteinander eingesetzt werden können, wie Cetyltrimethylammoniumchlorid, Dimethyldiethylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxy-ethyl)alkylammoniumphosphat, Cetylpyridiniumchlorid, etc. Gut geeignet sind auch die in der EP-A 472 107 geoffenbarten quaternären Ammoniumsalze.

Weitere geeignete langkettige Ammoniumverbindungen sind Esterquats der allgemeinen Formel (I) 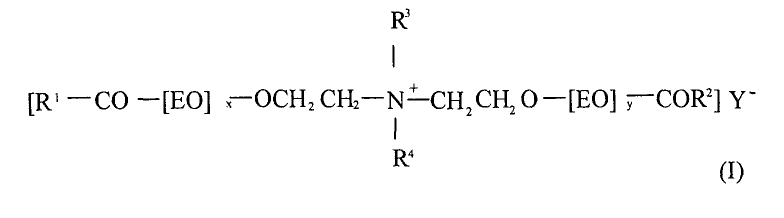 in der R¹ und R² für eine gegebenenfalls hydroxysubstituierte C₈-C₂₂-Alkyl-oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.

Eine besonders bevorzugte Verbindung der Formel I ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.

Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat^{R}", "Dehyquart^{R}F30" und "Tetranyl^{R}" im Handel.

Der Einsatz dieser "Esterquats" in Haarpflegemitteln ist an sich bekannt und beispielsweise in der WO-A 93/10748, der WO-A 92/06899 und der WO-A 94/16677 beschrieben.

Geeignet sind auch Amidoquats der allgemeinen Formel (II) 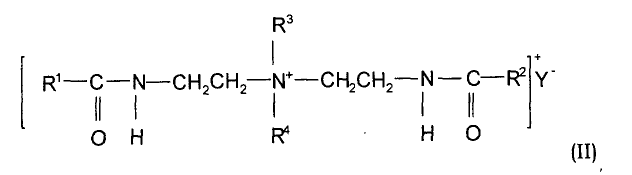 in der R¹ und R² jeweils für eine gegebenenfalls hydroxysubstituierte C₈-C₂₂-Alkyl-oder Alkenylgruppe, R³ und R⁴ für eine C₁-C₃-Alkylgruppe oder eine Gruppe-CH₂-CH₂-O-[EO]ₓ-H, sowie x für 0 bis 5, Y⁻ für ein Anion stehen.

Es können selbstverständlich auch Gemische aus den verschiedenen Tensiden, soweit sie miteinander kompatibel sind, verwendet werden.

Ein weiterer wünschenswerter Bestandteil der erfindungsgemäßen Färbemittel-Zusammensetzungen ist ein C₃-C₆-Alkandiol bzw. dessen Ether, insbesondere ein Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Färbemittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Färbemittels.

Diese Mittel können auch weitere Pflegemittel wie Öle und Fette enthalten. Solche sind beispielsweise Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.
Falls es sich bei den erfindungsgemäßen Mitteln um Emulsionen handelt, enthalten diese selbstverständlich die üblichen Emulgatoren.

Die erfindungsgemäßen Mittel können auch langkettige Fettsäuren enthalten.
Als Fettsäuren werden bevorzugt solche mit 10 bis 24, insbesondere 12 bis 22 Kohlenstoffatomen in einer Menge von etwa 0,5 bis 15 Gew.-%, insbesondere 1 bis 10 Gew.-%, bezogen auf die Gesamtzusammensetzung, verwendet. Besonders geeignet sind Behensäure und Stearinsäure; jedoch können auch andere Fettsäuren wie beispielsweise Myristinsäure, Palmitinsäure oder Ölsäure oder auch Gemische natürlicher oder synthetischer Fettsäuren wie Kokosfettsäure eingesetzt werden.

Die Viskosität der erfindungsgemäßen Mittel liegt vorzugsweise bei etwa 5000 bis etwa 60 000, insbesondere etwa 10 000 bis 50 000, vor allem etwa 20 000 bis 40 000 mPa.s bei 20°C, gemessen im Brookfield-Rotationsviskosimeter mit einer Spindel Nr. 5 bei 5rpm.

Der pH-Wert liegt vorzugsweise im sauren Bereich zwischen etwa 2 und 7, beispielsweise 2,5 und 6, insbesondere etwa 3 und 5,5.

Falls es sich um Tönungsshampoos handelt, können diese natürlich, neben den weiter oben aufgezählten oberflächenaktiven Substanzen, auch anionische Tenside enthalten.

Geeignete anionaktive Tenside sind in einer Menge von mindestens 5 bis etwa 50 Gew.-%, vorzugsweise 10 bis 25 Gew.-% der Zusammensetzung enthalten.

Dabei handelt es sich um solche vom Sulfat-, Sulfonat-, Carboxylat- und Alkylphosphat-Typ, vor allem natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.
Weiterhin geeignete anionische Tenside sind α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ-O-CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 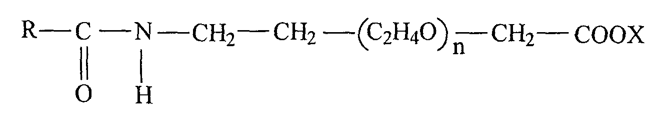 worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO® " und "AKYPO-SOFT® ".

Auch C₈-C₂₀-Acylisethionate können, allein oder im Gemisch mit anderen Tensiden, eingesetzt werden, ebenso Sulfofettsäuren und deren Ester.

Es können auch Mischungen aus mehreren anionischen Tensiden eingesetzt werden, beispielsweise ein Gemisch aus einem α-Olefinsulfonat und einem Sulfosuccinat, vorzugsweise im Verhältnis von 1 : 3 bis 3 : 1, oder einem Ethersulfat und einer Polyethercarbonsäure oder Alkylamidoethercarbonsäure.

Eine Übersicht über die in flüssigen Körperreinigungsmitteln zum Einsatz gelangenden anionaktiven Tenside findet sich im übrigen in der Monographie von K. Schrader, "Grundlagen und Rezepturen der Kosmetika", 2. Aufl. (1989, Hüthig Buchverlag), S. 683 bis 691.

Der bevorzugte Mengenbereich an anionischen Tensiden in Tönungsshampoos gemäß der Erfindung liegt zwischen etwa 5 und etwa 30 Gew.-%, insbesondere bei etwa 7,5 bis etwa 25 Gew.-%, besonders bevorzugt bei etwa 10 bis etwa 20 Gew.-%, berechnet auf die Gesamtzusammensetzung des Mittels, je nachdem, ob es sich um Konzentrate handelt.

Als bevorzugte Ausführungsform enthalten die erfindungsgemäßen Tönungsshampoos vorzugsweise ein Gemisch aus einem der genannten anionischen Tenside und mindestens einer C₈-C₂₂-Acylaminocarbonsäure bzw. deren wasserlöslichen Salzen, vorzugsweise in einer Menge von 0,5 bis 10, insbesondere 1 bis 7,5 Gew.-%, berechnet auf die Gesamtzusammensetzung.
Besonders bevorzugt ist das N-Lauroylglutamat, insbesondere als Natriumsalz. Weitere geeignete N-Acylaminocarbonsäuren sind beispielsweise N-Lauroyl-sarcosinat, N-C₁₂-C₁₈-Acylasparaginsäure, N-Myristoylsarcosinat, N-Oleoylsar-cosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze.

Die folgenden Beispiele dienen der Illustration der Erfindung.

### Beispiel 1

| | |
|---|---|
| Dimethicone Copolyol | 1,50 (Gew.-%) |
| Ethanol | 5,00 |
| Propylencarbonat | 25,00 |
| Milchsäure, 90%-ig | 5,00 |
| Natriumhydroxid, 32%-ig | 0,20 |
| Quaternäres Dimethylaminoethylmethacrylat-Homopolymerisat (Polyquaternium-37;50%-ig in einem Propylenglykoldicaprat-dicaprylat PPG-1-Trideceth-6 Gemisch; Salcare^{R}SC96) | 3,50 |
| Acid Orange 7 (C.I.-No. 15510) | 0,15 |
| Acid Yellow 3 (C.I.-No. 47005) | 0,10 |
| Acid Violet 43 (C.I.-No. 60 730) | 0,25 |
| Organopolysiloxan/Polyethyloxazolin-Pfropfcopolymerisat (Organopolysiloxan A1 der EP-A 640 643) | 0,10 |
| Wasser | ad 100,00 |
| pH-Wert: | 3,0 |

Die Zusammensetzung wurde auf nasses Haar aufgebracht.
Nach etwa zwanzigminütiger Einwirkung wurde gewaschen, gespült und getrocknet. Es wurde ein glänzend und gleichmaßig mittelbraun gefärbtes Haar, das leicht kämmbar war, einen vollen Griff und Spannkraft aufwies, erhalten. Die Färbung blieb über vier Haarwäschen haltbar.

Weglassen des Organopolysiloxans führte zu einem sowohl in seiner Konditionierung als auch insbesondere hinsichtlich der Farbstabilität und -intensivität des Glanzes sowie der Gleichmaßigkeit der Färbung deutlich unterlegenem Haar.

### Beispiel 2

| | |
|---|---|
| Vinylpyrrolidon/Vinylacetat-Copolymer (Luviskol^{R}VA64W) | 1,60 (Gew.-%) |
| Xanthan-Gum (Keltrol^{R}) | 1,50 |
| Parfum | 0,30 |
| Phenoxyethanol | 0,10 |
| 1,2-Propandiol | 0,50 |
| Ethanol | 0,75 |
| Basic Blue 99 (C.I.-No 56 059) | 0,10 |
| Basic Brown 16 (C.I.-No. 12 250) | 0,18 |
| Basic Brown 17 (C.I.-No. 12 251) | 0,32 |
| Basic Yellow 57 (C.I.-No. 12 719) | 0,36 |
| Organopolysiloxan/Polyethyloxazolin-Pfropfcopolymerisat (Organopolysiloxan A1 der EP-A 640 643) | 1,00 |
| | |
| Wasser | ad 100,00 |
| pH-Wert: | 5,8 |

Bei der Haarfärbung entsprechend dem in Beispiel 1 beschriebenen Verfahren wurde eine glänzende, über mehrere Haarwäschen stabile, gleichmäßige Goldfärbung erzielt.
Das Haar wies ausgezeichnete Konditioniereigenschaften auf.

Ersatz des Organopolysiloxans durch die gleiche Menge Polyquaternium-7 führte zu einer schwächeren, ungleichmaßigen Anfärbung mit geringem Glanz und reduzierter Licht- und Haarwaschstabilität.

### Beispiel 3

| | |
|---|---|
| Polyquaternium-6 | 0,60 (Gew.-%) |
| Cetrimoniumchlorid | 0,80 |
| Ethanol | 7,40 |
| Parfum | 0,30 |
| PVP/VA-Copolymer | 0,70 |
| Basic Blue 99 (C.I.-No 56059) | 0,04 |
| Basic Brown 16 (C.I.-No. 12250) | 0,36 |
| HC-Red 3 | 0,17 |
| HC-Yellow 5 | 0,33 |
| Organopolysiloxan/Polyethyloxazolin-Pfropfcopolymerisat (Organopolysiloxan A1 der EP-A 640 643) | 0,50 |
| | |
| Wasser | ad 100,00 |
| pH-Wert: | 5,8 |

Bei der Haarfärbung wurde ein glänzender, gleichmäßiger Kupferrotton erhalten, der nach fünf Haarwäschen noch deutlich war.

Weglassen des Organopolysiloxans führte zu einer weniger intensiven, bereits nach drei Haarwäschen verblassenden Haarfärbung.

### Beispiel 4

Ein Tönungsshampoo der Zusammensetzung

| | |
|---|---|
| Natrium-C₁₂-C₁₄-alkylethersulfat (2-3 EO) | 10,00 (Gew.-%) |
| Cocoamidopropylbetain | 2,00 |
| PEG-3-distearat | 2,00 |
| Organopolysiloxan/Polyethyloxazolin-Pfropfcopolymerisat (Organopolysiloxan A1 der EP-A 640 643) | 1,00 |
| | |
| Basic Red 2 | 0,15 |
| Citronensäure, Konservierungsmittel | q.s. |
| Wasser | ad 100,00 |

wurde auf das Haar aufgebracht und nach zehnminütiger Einwirkung gespült und getrocknet.

Es wurde eine ausdrucksvolle, intensive, dauerhafte, gleichmäßige glänzende Rotfärbung erzielt, die nach drei Haarwäschen noch in gutem Zustand war.

Weglassen des Organopolysiloxans führte zu einer wesentlich schwächeren Färbung, die bereits nach einer Haarwäsche verblaßt war.

### Beispiel 5

Menschliches Haar wurde mit einem Tönungsshampoo der Zusammensetzung

| | |
|---|---|
| C₁₂-C₁₄-Alkylpolyglucosid (P.D.∼1,45) | 10,00 (Gew.-%) |
| Cocoamidopropylbetain | 2,00 |
| PEG-3-distearat | 2,00 |
| Organopolysiloxan/Polyethyloxazolin-Pfropfcopolymerisat (Organopolysiloxan A1 der EP-A 640 643) | 1,50 |
| | |
| Parfum | 0,40 |
| Citronensäure, Konservierungsmittel | q.s. |
| Basic Brown 16 | 0,10 |
| Basic Brown 17 | 0,10 |
| Basic Blue 99 | 0,02 |
| Wasser | ad 100,00 |

gewaschen, gespült und getrocknet.
Es wurde eine ausdrucksvolle, glänzende, dauerhafte, intensive, gleichmäßige Braunfärbung erzielt, die auch nach drei Haarwäschen noch in gutem Zustand war.

Weglassen der Verbindung des Organopolysiloxans führte zu einem Produkt, das eine matte Braunfärbung ergab, die nach nur einer Haarwäsche total verblaßt war.

Eine Konditionierwirkung konnte mit diesem Produkt ebenfalls nicht erzielt werden.

## Patentansprüche

1. Mittel zum Färben von menschlichen Haaren, enthaltend
a) mindestens einen direktziehenden Haarfarbstoff, und
b) mindestens ein Organopolysiloxan, bei dem mindestens ein Siliciumatom über eine Alkylengruppe, die ein Heteroatom, insbesondere ein Stickstoffatom, aufweist, mit einem Poly-(N-acylalkylenimin) mit Einheiten der Formel I 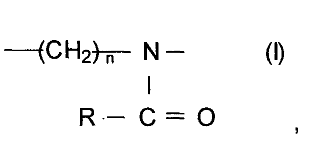 wobei n eine Zahl von 1 bis 5 und R Wasserstoff, eine C₁-C₁₂-Alkyl-, Cycloalkyl-, Aralkyl- oder Arylgruppe bedeuten, verbunden ist.

2. Mittel nach Anspruch 1, enthaltend als Organosiloxan ein gegebenenfalls quaterniertes Aminoalkyldimethylpolysiloxan/Polyethyloxazolin-Copolymerisat der Formel 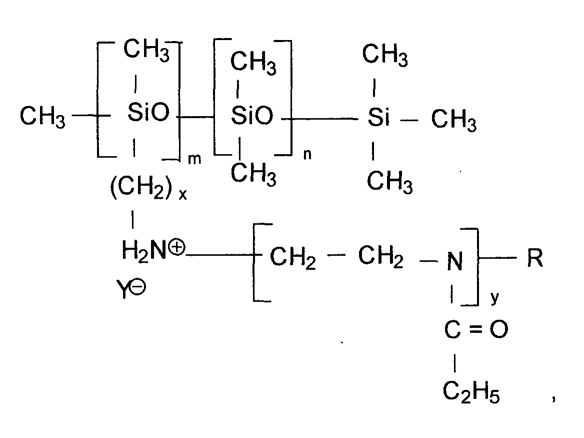 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R eine C₁-C₁₂-Alkyl- oder Arylgruppe, insbesondere eine Methyl-, Ethyl- oder Benzylgruppe, und Y⁻ ein Anion darstellen.

3. Mittel nach Anspruch 1 oder 2, enthaltend 0,05 bis 5 Gew.-%, berechnet auf die Gesamtzusammensetzung, des Organopolysiloxans.

4. Mittel nach Anspruch 3, enthaltend 0,1 bis 2,5 Gew.-% des Organopolysiloxans, berechnet auf die Gesamtzusammensetzung des Mittels.

5. Verwendung eines Organopolysiloxans der Formel I 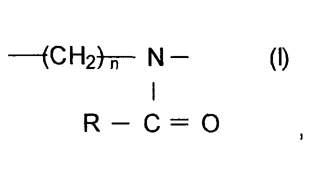 wobei n eine Zahl von 1 bis 5 und R Wasserstoff, eine C₁-C₁₂-Alkyl-, Cycloalkyl-, Aralkyl- oder Arylgruppe bedeuten, verbunden ist, in Mitteln zum Färben von menschlichen Haaren, die mindestens einen direktziehenden Haarfarbstoff enthalten.
